# EUROPÄISCHE PATENTSCHRIFT

(11) **EP 1 768 720 B1**
(45) Veröffentlichungstag und Bekanntmachung des Hinweises auf die Patenterteilung: **20.02.2013**
(21) Anmeldenummer: 05762741.6
(22) Anmeldetag: 14.06.2005
(51) Int. Cl.: A61M 1/02, B01D 19/00, A61M 5/165

(54) **GESCHLOSSENES STERILES SYSTEM UND VERFAHREN ZUM FILTRIEREN VON BIOLOGISCHEN ODER MEDIZINISCHEN FLÜSSIGKEITEN, INSBESONDERE VON VOLLBLUT**
SEALED, STERILE SYSTEM AND METHOD FOR FILTERING BIOLOGICAL OR MEDICAL FLUIDS, IN PARTICULAR WHOLE BLOOD
SYSTEME STERILE FERME ET PROCEDE POUR FILTRER DES LIQUIDES BIOLOGIQUES OU MEDICAUX, NOTAMMENT LE SANG TOTAL

(30) Priorität: 21.07.2004 DE 102004035352
(43) Veröffentlichungstag der Anmeldung: 04.04.2007
(60) Teilanmeldung: 12189286.3
(73) Patentinhaber: Fresenius HemoCare Austria GmbH, 5301 Eugendorf (AT)
(72) Erfinder: SOMMER, Christian, AT-4810 Gmunden (AT); KURZ, Michael, AT-5400 Hallein-Rif (AT); MAYER, Günter, AT-5023 Salzburg (AT)
(74) Vertreter: Mai, Oppermann & Partner i.L.
(86) Internationale Anmeldenummer: PCT/EP2005/006338
(87) Internationale Veröffentlichungsnummer: WO 2006/007906

(56) Entgegenhaltungen:
- EP-A- 0 888 789
- WO-A-01/76721
- WO-A-98/39080
- US-A- 5 776 338
- US-A1- 2002 011 452

## Beschreibung

Die Erfindung betrifft ein geschlossenes steriles System zum Filtrieren von biologischen oder medizinischen Flüssigkeiten, insbesondere von Vollblut sowie ein Verfahren zum Filtrieren von biologischen oder medizinischen Flüssigkeiten, insbesondere Vollblut.

Die EP 0 349 188 B1 beschreibt eine Vorrichtung zum Trennen von Blut in Blutkomponenten, die einen Blutsammelbeutel aufweist, der über eine erste Schlauchleitung mit einem Primärbeutel verbunden ist, wobei der Primärbeutel über eine weitere Schlauchleitung mit einem Satellitenbeutel verbunden ist. In der ersten Schlauchleitung, die den Blutsammelbeutel mit dem Primärbeutel verbindet, ist ein Filter zur Entfernung von Leukozyten angeordnet. Zur Gewinnung von leukozytenfreien Blutkomponenten wird Vollblut eines Spenders in dem Blutsammelbeutel gesammelt und über die den Filter enthaltende Schlauchleitung in den Primärbeutel überführt. Daraufhin wird der zwischen dem Filter und dem Primärbeutel liegende Abschnitt der Schlauchleitung durchtrennt, wobei die Trennstelle abgedichtet wird. Der Primärbeutel wird dann zusammen mit dem Sekundärbeutel zentrifugiert, um das in dem Primärbeutel befindliche Blut in zwei Blutkomponenten aufzutrennen. Eine der beiden Blutkomponenten wird dann über die zweite Schlauchleitung in den Satellitenbeutel überführt. Auf diese Weise können nach der Filtration des Vollblutes leukozytenfreie Blutkomponenten gewonnen werden. Ein entscheidender Vorteil wird bei dem bekannten Verfahren darin gesehen, dass eine Zentrifugation der Beutelanordnung ohne den Filter erfolgt und eine mechanische Beanspruchung des Filters somit vermieden wird.

Die aus der EP 0 349188 B1 bekannte Vorrichtung zum Filtrieren von Vollblut ist insofern nachteilig, als nach der Beendigung des Filtrationsvorganges, d.h. nachdem das Vollblut aufgehört hat unter der Wirkung der Schwerkraft von dem Blutsammelbeutel in den Primärbeutel zu fließen, noch Flüssigkeit in de Leukozytenfilter und den betreffenden Schlauchleitungsabschnitten verbleiben kann.

Die WO 91/17809 beschreibt ein Filtriersystems, das einen Sammelbeutel zur Aufnahme der filtrierten Flüssigkeit umfasst, der über eine Schlauchleitung, in der ein Filter angeordnet ist, mit einem die zu filtrierende Flüssigkeit enthaltenden Vorlagebeutel verbunden ist. Die zu filtrierende Flüssigkeit wird unter dem Einfluss der Schwerkraft aus dem Vorlagebeutel über die Schlauchleitung und den Filter in den Sammelbeutel überführt. Zur Rückgewinnung der nach Beendigung des Filtrationsvorganges in dem Filter und den angrenzenden Schlauchleitungsabschnitten verbleibenden Flüssigkeit ist stromauf des Filters an der Schlauchleitung ein Gasein- und stromab des Filters ein Gasauslass vorgesehen. Nach Beendigung des Filtrationsvorganges wird über den Einlass in das System Gas geleitet, das unter Verdrängung der in dem System befindlichen Flüssigkeit, wieder über den Auslass abgeführt wird. Bei einer bevorzugten Ausführungsform des bekannten Filtriersystems wird das nach Beendigung des Filtrationsvorganges in das System zu leitende Gas in einem flexiblen Beutel bereitgestellt, der über eine Schlauchleitung an dem Gaseinlass angeschlossen ist. Die aus dem System abgeführte Luft wird in einem zweiten flexiblen Beutel gesammelt, der über eine zweite Schlauchleitung an dem Gasauslass angeschlossen ist. Zur Rückgewinnung der in dem System befindlichen Flüssigkeit wird nach Beendigung des Filtrationsvorganges der erste Beutel zusammengepresst, wodurch das Gas in das System unter Verdrängung der Flüssigkeit strömt. Nachteilig ist, dass das bekannte Filtriersystems mit dem Gasein- und -auslass relativ aufwendig herzustellen und schwer zu handhaben ist.

Aus der WO 03/064000 A1 ist ebenfalls eine Filtriervorrichtung bekannt, die einen Filter umfasst. Die Filtriervorrichtung weist eine Schlauchleitung auf, die einen Vorlagebeutel mit einem Sammelbeutel verbindet und in die ein Filter geschaltet ist, das von einem Filtermaterial in eine erste und zweite Kammer unterteilt ist. Darüber hinaus weist die Vorrichtung einen weiteren Beutel auf, der mit der ersten und zweiten Filterkammer jeweils über eine Lufteinlass- bzw. Auslassleitung in Strömungsverbindung steht. In einer der beiden Leitungen ist ein Rückschlagventil angeordnet, das einen Durchlass von Fluid nur in Richtung der ersten Filterkammer erlaubt. Der Beutel dient der Entlüftung des Systems beim Überführen der Flüssigkeit aus dem Vorlagebeutel in den Sammelbeutel. Nachteilig ist, dass die Einschaltung des Beutels mit den Lufteinlass- bzw. Auslassleitungen die Handhabung des Systems erschwert.

Die WO 01/76721 Al beschreibt ein geschlossenes steriles System, das einen ersten und einen zweiten kollabierbaren Behälter zur Aufnahme von Flüssigkeit umfasst, die über eine Schlauchleitung miteinander verbunden sind, in der ein Filter angeordnet ist. An dem zweiten kollabierbaren Behälter ist über eine weitere Schlauchleitung ein dritter kollabierbarer Behälter angeschlossen, der zur Entlüftung des Systems dient.

Der Erfindung liegt die Aufgabe zugrunde, ein geschlossenes steriles System zum Filtrieren von biologischen und medizinischen Flüssigkeiten, insbesondere Vollblut, bereitzustellen, das sich einfach handhaben und mit relativ geringen Kosten herstellen lässt. Darüber hinaus ist eine Aufgabe der Erfindung, ein einfach durchzuführendes Verfahren zum Filtrieren von biologischen oder medizinischen Flüssigkeiten anzugeben.

Die Lösung dieser Aufgaben erfolgt erfindungsgemäß mit den Merkmalen der Patentansprüche 1 und 9.

Bei dem erfindungsgemäßen geschlossenen sterilen Filtrationssystem und dem Verfahren wird die Luft, die beim Überführen der zu filtrierenden Flüssigkeit von dem ersten kollabierbaren Behälter durch den Filter in den zweiten kollabierbaren Behälter für die vollständige Entleerung des Filters bzw. der angrenzenden Schlauchleitungsabschnitte erforderlich ist, in dem ersten kollabierbaren Behälter bereitgestellt. Dadurch unterscheidet sich das erfindungsgemäße Filtriersystem und Verfahren grundlegend von den bekannten System und Verfahren, bei denen der Beutel zur Aufnahme von Vollblut vollständig mit einem Antikoagulanz befüllt wird. Folglich entfällt bei der erfindungsgemäßen Vorrichtung und dem Verfahren die ansonsten erforderliche Absaugung der Luft, die sich in dem mit Antikoagulanz befüllten Blutsammelbeutel befindet.

Um eine vollständige Entlüftung zu gewährleisten, sollte die vorgegebene Menge an Luft größer als das Volumen des Filters sowie der angrenzenden Schlauchleitungsabschnitte sein. Neben der in dem ersten Behälter befindlichen Luft kann auch die in den angrenzenden Leitungsabschnitten befindliche Luft zur Entlüftung des Systems verwandt werden.

Die in dem ersten kollabierbaren Behälter vorgegebene Menge an Luft wird zusammen mit der zu filtrierenden Flüssigkeit in den zweiten kollabierbaren Behälter überführt, während sich der Filter und die angrenzenden Schlauchleitungsabschnitte vollständig entleeren. Ein besonderer Vorteil liegt darin, dass zur Überführung der Flüssigkeit nur verhältnismäßig geringe Kräfte erforderlich sind. Die Überführung der Flüssigkeit kann unter dem Einfluss der Schwerkraft des ersten kollabierbaren Behälters erfolgen. Weitere Schritte sind zum Überführen der Flüssigkeit nicht erforderlich.

Das erfindungsgemäße Filtriersystem verfügt über einen dritten kollabierbaren Behälter, dessen Füllvolumen kleiner als das Füllvolumen des ersten oder zweiten kollabierbaren Behälters ist. Nach dem Überführen der Flüssigkeit aus dem ersten in den zweiten kollabierbaren Behälter kann die in dem zweiten kollabierbaren Behälter befindliche Luft in den dritten kollabierbaren Behälter überführt werden. Damit ist der zweite kollabierbare Behälter vollständig entlüftet. Der dritte kollabierbare Behälter kann aber auch zur Probenentnahme dienen, wenn auch ein Teil der in dem dritten Behälter befindlichen Flüssigkeit in den zweiten Behälter überführt wird.

Das Füllvolumen des dritten kollabierbaren Behälters sollte größer als das Volumen des Filters sowie der angrenzenden Schlauchabschnitte sein, so dass die zur Entlüftung erforderliche Menge an Luft von dem zweiten Behälter vollständig in den dritten Behälter überführt werden kann.

Bei einer bevorzugten Ausführungsform der Erfindung ist der erste kollabierbare Behälter ein Sammelbeutel für Vollblut, der vorzugsweise bis auf die vorgegebene Menge an Luft mit einer Flüssigkeit, insbesondere einem Antikoagulanz befüllt ist.

Zum Sammeln der zu filtrierenden Flüssigkeit weist der erste kollabierbare Behälter vorzugsweise einen Einlass auf, zu dem eine öffenbare Sammelleitung führt. Vorzugsweise ist die öffenbare Sammelleitung eine Blutentnahmeleitung mit einer Nadel, die von einer Schlauchklemme verschlossen ist.

Eine weitere bevorzugte Ausführungsform sieht neben den drei Behältern noch eine Satellitenbehältnisanordnung vor, die ein oder mehrere Satellitenbehältnisse enthält. Die Satellitenbehältnisanordnung erlaubt die Auftrennung des gefilterten Vollblutes in weitere Blutkomponenten nach den bekannten Verfahren.

Die kollabierbaren Behälter sind vorzugsweise Beutel aus flexiblen Folien. Derartige Beutel gehören zum Stand der Technik.

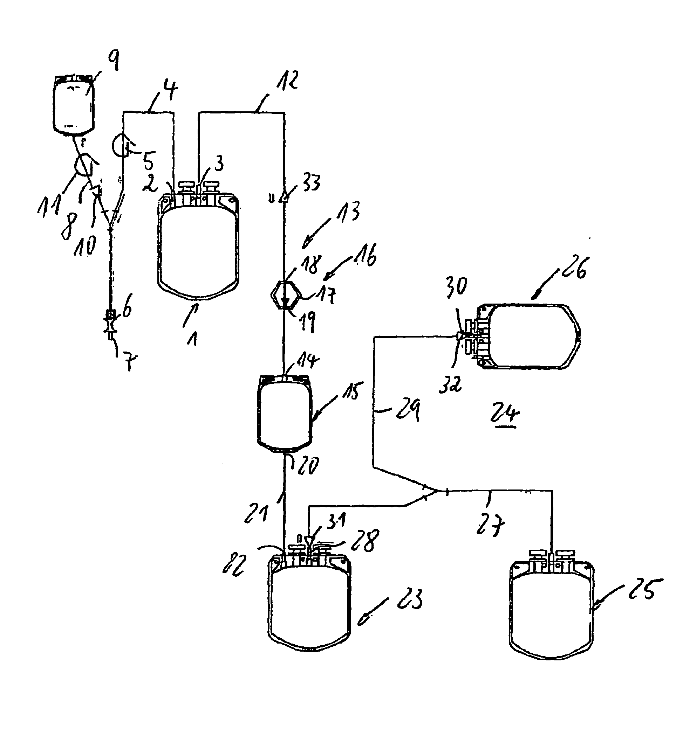

Im folgenden wird ein Ausführungsbeispiel des erfindungsgemäßen sterilen geschlossenen Filtriersystems sowie das erfindungsgemäße Filtrierverfahren unter Bezugnahme auf die Zeichnung näher erläutert, die das erfindungsgemäße Filtriersystem in schematischer Darstellung zeigt.

Das geschlossene sterile System zum Filtrieren von Vollblut umfasst einen ersten kollabierbaren Behälter 1, der einen Einlass 2 und einen Auslass 3 aufweist. Bei dem kollabierbaren Behälter handelt es sich um einen konventionellen Beutel aus einer flexiblen Folie. Auch bei den weiteren Behältern, die das System umfasst, handelt es sich bei dem Ausführungsbeispiel um Folienbeutel.

An dem Einlass 2 des Blutsammelbeutels 1 ist eine Blutentnahmeleitung 4 angeschlossen, die von einer Schlauchklemme 5 verschlossen ist. An dem freien Ende der Blutentnahmeleitung 4 ist über einen Adapter 6 eine Nadel 7 angeschlossen, die zur Entnahme des Vollbluts von dem Spender dient. Von der Blutentnahmeleitung 4 zweigt zwischen dem Adapter 6 mit der Nadel 7 und der Schlauchklemme 5 eine Zuführleitung 8 ab, die zu dem Auslass eines ersten Probenentnahmebeutels 9 führt. Die Zuführleitung 8 ist mit einem Abbrechteil 10 verschlossen, der es erlaubt, durch Abbrechen eines Ventilstücks eine Strömungsverbindung herzustellen. Derartige Abbrechteile gehören zum Stand der Technik. Darüber hinaus ist die Zuführleitung 8 mit einer Schlauchklemme 11 verschlossen, die es erlaubt, die Strömungsverbindung auch nach dem Abbrechen des Ventilstücks des Abbrechteils 10 zu unterbrechen.

Von dem Auslass 3 des Blutsammelbeutels 1 geht ein erster Abschnitt 12 einer Vollblutleitung 13 ab, die zu einem Einlass 18 eines Leukozytenfilters 16 zum Filtrieren des Vollblutes führt, der ein Gehäuse 17 aufweist, das von einem Filtermaterial in zwei nicht dargestellte Kammern unterteilt ist. Von einem Auslass 19 des Filters 17 geht ein zweiter Abschnitt 21 der Blutleitung 13 ab, der zu einem Einlass eines Beutels 23 zur Aufnahme des filtrierten Vollbluts führt. In dem zweiten Abschnitt 21 der Blutleitung 13 ist ein Entlüftungsbeutel 15 angeordnet, der einen Einlass 14 und Auslass 20 aufweist.

Das sterile System umfasst weiterhin eine Satellitenbehältnisanordnung 24, die ein oder mehrere Satellitenbehältnisse enthält. Bei dem vorliegenden Ausführungsbeispiel enthält die Satellitenbehältnisanordnung 24 einen Satellitenbeutel 25 und einen Zusatzmittelbeutel 26, der mit einer Additivlösung gefüllt ist. Der erste Satellitenbeutel 25 ist über eine Satellitenleitung 27 an einem Auslass 28 des Beutels 23 angeschlossen. Von der Satellitenleitung 27 zweigt eine weitere Überführungsleitung 29 ab, die zu einem Einlass/Auslass 30 des Beutels 26 führt. Die Satelliten- und Überführungsleitung 27 und 29 sind wieder mit den bekannten Abbrechteilen 31, 32 verschlossen, so dass vor dem Abbrechen der Abbrechteile die Satellitenbehältnisanordnung 24 von dem Beutel 23 zur Aufnahme der filtrierten Flüssigkeit getrennt sind.

Der Entlüftungsbeutel 15 hat ein Füllvolumen, das kleiner als das Füllvolumen des Blutsammelbeutels 1 oder des Beutels 23 zur Aufnahme des filtrierten Bluts ist. Das Füllvolumen des Entlüftungsbeutels 15 ist auf jeden Fall größer als das Volumen, was von den beiden Kammern des Filters 16, dem ersten und zweiten Abschnitt 12, 21 der Blutleitung 13, der Blutentnahmeleitung 4 und dem Abschnitt der Zuführleitung 8 stromab des Abbrechteils 10 eingeschlossen ist.

Zwischen dem Blutsammelbeutel 1 und dem Filter 16 ist ein weiterer Abbrechteil 33 angeordnet, der die Blutleitung 13 verschließt. Die Nadel 7 ist vor Gebrauch des Filtriersystems steril verschlossen, so dass die Anordnung ein steriles geschlossenes System bildet.

Der Blutsammelbeutel 1 ist bis auf eine vorgegebene Menge an Luft mit einem Antigerinnungsmittel (Antikoagulanz) befüllt. Bei der Fertigung ist der erste Abschnitt 12 der Blutleitung 13 zunächst durchtrennt. Das Antikoagulanz wird über den mit dem Blutsammelbeutel 1 verbundenen Abschnitt 12 der Blutleitung 13 befüllt. Bei der Füllung lässt man die Flüssigkeitssäule bis knapp vor den Einlass des Beutels 1 zurückfließen, bevor die Abschnitte der Blutleitung 13 über den Abbrechteil 33 miteinander verbunden werden. Da auf Vakuumsaugen bei der Füllung verzichtet wird, verbleibt neben der Luftblase in dem Beutel 1 noch Luft in den Schlauchleitungen. Der Entlüftungsbeutel 15 enthält vorzugsweise keine Luft. Auch die anderen Beutel enthalten vorzugsweise keine Luft.

Zur Entnahme von Vollblut wird nach der Punktion die Schlauchklemme 5 geöffnet, so dass das Vollblut von dem Spender in den mit dem Antikoagulanz befüllten Blutsammelbeutel 1 strömt. Dabei schiebt das Blut die in den betreffenden Schlauchleitungsabschnitten vorhandene Luft in den Blutsammelbeutel 1.

Anschließend wird das Abbrechstück des Abbrechteils 33 abgebrochen, so dass eine Strömungsverbindung zwischen dem Blutsammelbeutel 1 und dem Beutel 23 zur Aufnahme des filtrierten Vollblutes hergestellt ist. Das Vollblut wird nun zusammen mit der in dem Beutel 1 befindlichen Luft durch den Filter 16 über den Entlüftungsbeutel 15 in den Beutel 23 überführt. Da die in dem Beutel 1 befindliche Luft ausreichend ist, um das System zu entlüften, verbleibt in den beiden Kammern des Filters 16 und den betreffenden Schlauchleitungsabschnitten sowie dem Entlüftungsbeutel 15 keine Restflüssigkeit. In dem Beutel 23 befindet sich jetzt das von Leukozyten befreite Vollblut. Die Überführung des Vollblutes kann allein unter Schwerkraft erfolgen.

Die in dem Beutel 23 befindliche Restluft kann nun in den Entlüftungsbeutel 15 überführt werden. Dazu wird der Beutel 23 zusammengedrückt, so dass die Luft über den zweiten Abschnitt 21 der Blutleitung 13 in den Beutel 15 gelangt. Der Entlüftungsbeutel 15 kann nicht nur zur Entlüftung des Systems, sondern auch zur Entnahme von Proben für Qualitätskontrollen dienen. Der Entlüftungsbeutel 15 hat bei dem Ausführungsbeispiel ein Volumen von 60 ml.

Daraufhin kann der das leukozytenfreie Vollblut enthaltende Beutel 23 zusammen mit der Satellitenbehältnisanordnung 24 von den übrigen Komponenten des Filtriersystems abgetrennt werden, um in bekannter Weise durch Zentrifugieren aus dem Vollblut einzelne Blutkomponenten zu gewinnen.

## Patentansprüche

1. Ein geschlossenes steriles System zum Filtrieren von Flüssigkeiten, insbesondere von Vollblut, mit
einem ersten kollabierbaren Behälter (1) zur Aufnahme der zu filtrierenden Flüssigkeit,
einem Filter (16), der ein Gehäuse (17) aufweist, das durch ein Filtermaterial in zwei Kammern unterteilt ist, von denen die erste Kammer mit einem Einlass (18) und die zweite Kammer mit einem Auslass (19) versehen ist,
einem zweiten kollabierbaren Behälter (23) zur Aufnahme der gefilterten Flüssigkeit und
einem von einem Auslass (3) des ersten kollabierbaren Behälters (1) zu dem Einlass (18) des Filters führenden ersten Abschnitt einer
Flüssigkeitsleitung und von einem von dem Auslass des Filters (19) zu einem Einlass (22) des zweiten kollabierbaren Behälters (23) führenden
zweiten Abschnitt (21) der Flüssigkeitsleitung (13),
**dadurch gekennzeichnet,**
**dass** der erste kollabierbare Behälter (1) eine vorgegebene Menge an Luft enthält, und dass in dem zweiten Abschnitt (21) der Flüssigkeitsleitung (22) ein dritter kollabierbarer Behälter (15) zum Entlüften des zweiten kollabierbaren Behälters (23) angeordnet ist, wobei das Füllvolumen des dritten kollabierbaren Behälters (15) kleiner als das Füllvolumen des ersten oder zweiten kollabierbaren Behälters (1, 23) ist.

2. Steriles System nach Anspruch 1,
**dadurch gekennzeichnet, dass** der erste kollabierbare Behälter (1) bis auf die vorgegebene Menge an Luft mit einer Flüssigkeit befüllt ist.

3. Steriles Systems nach Anspruch 1 oder 2,
**dadurch gekennzeichnet, dass** der erste kollabierbare Behälter (1) einen Einlass (2) aufweist, zu dem eine öffenbare Sammelleitung (4) führt.

4. Steriles System nach einem der Ansprüche 1 bis 3,
**dadurch gekennzeichnet, dass** der zweite kollabierbare Behälter (23) einen Auslass (28) aufweist, von dem eine Satellitenleitung (27) zu einer Satellitenbehältnisanordnung (24) führt, die ein oder mehrere Satellitenbehältnisse (25) enthält.

5. Steriles System nach einem der Ansprüche 1 bis 4,
**dadurch gekennzeichnet, dass** der erste kollabierbare Behälter (1) ein Sammelbeutel für Vollblut ist.

6. Steriles Systems nach Anspruch 5,
**dadurch gekennzeichnet, dass** der Blutsammelbeutel (1) mit einem Antikoagulanz befüllt ist.

7. Steriles Systems nach einem der Ansprüche 1 bis 6,
**dadurch gekennzeichnet, dass** der Filter ein Leukozytenfilter (16) ist.

8. Steriles System nach einem der Ansprüche 3 bis 7,
**dadurch gekennzeichnet, dass** die Sammelleitung (4) eine Blutentnahmeleitung mit einer Nadel (7) ist, die von einer Schlauchklemme (5) verschlossen ist.

9. Verfahren zum Filtrieren von Flüssigkeiten, insbesondere von Vollblut, mit einer Vorrichtung nach einem der Ansprüche 1 bis 8, **gekennzeichnet durch** die folgenden Verfahrensschritte:
Sammeln der zu filtrierenden Flüssigkeit, insbesondere des Vollblutes, in dem ersten kollabierbaren Behälter (1), der eine vorgegebene Menge von Luft enthält,
Überführen der Flüssigkeit zusammen mit der vorgegebenen Menge an Luft von dem ersten kollabierbaren Behälter (1) **durch** den Filter (16) und den dritten kollabierbaren Behälter (15) in den zweiten kollabierbaren Behälter (23) und
Überführen der vorgegebenen Menge an Luft von dem zweiten kollabierbaren Behälter (23) in den dritten kollabierbaren Behälter (15), der ein Füllvolumen hat, das kleiner als das Füllvolumen des ersten oder zweiten kollabierbaren Behälters ist.

10. Verfahren nach Anspruch 9,
**dadurch gekennzeichnet, dass** die zu filtrierende Flüssigkeit Vollblut ist.

11. Verfahren nach Anspruch 9 oder 10,
**dadurch gekennzeichnet, dass** der Filter (16) ein Leukozytenfilter ist.

12. Verfahren nach einem der Ansprüche 9 bis 11,
**dadurch gekennzeichnet, dass** vor dem Sammeln von Vollblut der erste kollabierbare Behälter (1) bis auf die vorgegebene Menge an Luft mit einem Antikoagulanz befüllt wird.

## Claims

1. A closed sterile system for the filtration of fluids, in particular of full blood, comprising
a first collapsible container (1) for accommodating the fluid to be filtered,
a filter (16) which comprises a housing (17) which is divided by a filter material into two chambers, whereof the first chamber is provided with an inlet (18) and the second chamber with an outlet (19),
a second collapsible container (23) for accommodating the filtered fluid and
a first segment of a fluid line leading from an outlet (3) of the first collapsible container (1) to the inlet (18) of the filter and a second segment (21) of the fluid line (13) leading from the outlet of the filter (19) to an inlet (22) of the second collapsible container (23),
**characterised in that**
the first collapsible container (1) contains a predetermined quantity of air, and that a third collapsible container (15) for venting the second collapsible container (23) is disposed in the second segment (21) of the fluid line (22), wherein the filling volume of the third collapsible container (15) is smaller than the filling volume of the first or second collapsible container (1, 23).

2. The sterile system according to claim 1,
**characterised in that** the first collapsible container (1) is filled with a fluid up to the predetermined quantity of air.

3. The sterile system according to claim 1 or 2,
**characterised in that** the first collapsible container (1) comprises an inlet (2), to which an openable collecting line (4) leads.

4. The sterile system according to any one of claims 1 to 3, **characterised in that** the second collapsible container (23) comprises an outlet (28), from which a satellite line (27) leads to a satellite container arrangement (24), which contains one or more satellite containers (25).

5. The sterile system according to any one of claims 1 to 4,
**characterised in that** the first collapsible container (1) is a collecting bag for full blood.

6. The sterile system according to claim 5,
**characterised in that** the blood collecting bag (1) is filled with an anticoagulant.

7. The sterile system according to any one of claims 1 to 6,
**characterised in that** the filter is a leucocyte filter (16).

8. The sterile system according to any one of claims 3 to 7,
**characterised in that** the collecting line (4) is a blood removal line with a needle (7), which is closed by a hose clamp (5).

9. A method for the filtration of fluids, in particular full blood, with a device according to any one of claims 1 to 8, **characterised by** the following method steps:
collection of the fluid to be filtered, in particular the full blood, in the first collapsible container (1), which contains a predetermined quantity of air,
transfer of the fluid together with the predetermined quantity of air from the first collapsible container (1) through the filter (16) and the third collapsible container (15) into the second collapsible container (23) and
transfer of the predetermined quantity of air from the second collapsible container (23) into the third collapsible container (15), which has a filling volume which is smaller than the filling volume of the first or second collapsible container.

10. The method according to claim 9,
**characterised in that** the fluid to be filtered is full blood.

11. The method according to claim 9 or 10,
**characterised in that** the filter (16) is a leucocyte filter.

12. The method according to any one of claims 9 to 11, **characterised in that**, before the collection of full blood, the first collapsible container (1) is filled with an anticoagulant up to the predetermined quantity of air.

13. A system for filtering a fluid, comprising a filter, a hose line which connects a storage bag to a collecting bag and into which the filter is incorporated, wherein the filter is divided into a first and second chamber, a further bag which is in a flow connection with the first and second filter chamber in each case via an air inlet and outlet line, there being disposed in one of the two lines a non-return valve which permits the passage of the fluid only in the direction of the first filter chamber, wherein the bag is used to vent the system during the transfer of fluid from the storage bag into the collecting bag.

## Revendications

1. Système stérile fermé pour filtrer des liquides, notamment le sang total, avec :
(i) un premier récipient comprimable (1) destiné à contenir le liquide à filtrer ;
(ii) un filtre (16), qui présente un boîtier (17), qui est divisé en deux chambres par un matériau de filtrage, dans lesquelles la première chambre est munie d'une entrée (18) et la deuxième chambre est munie d'une sortie (19) ;
(iii) un deuxième récipient comprimable (23) destiné à contenir le liquide filtré ; et
(iv) une première partie d'une conduite de liquide menant d'une sortie (3) du premier récipient comprimable (1) à l'entrée (18) du filtre et une deuxième partie (21) de la conduite de liquide (13) menant de la sortie du filtre (19) à une entrée (22) du deuxième récipient comprimable (23),
**caractérisé en ce que** le premier récipient comprimable (1) contient une quantité prédéterminée d'air, et **en ce qu'**un troisième récipient comprimable (15) est disposé dans la deuxième partie (21) de la conduite de liquide (22) pour purger l'air du deuxième récipient comprimable (23), dans lequel le volume de remplissage du troisième récipient comprimable (15) est plus petit que le volume de remplissage du premier ou du deuxième récipient comprimable (1, 23).

2. Système stérile selon la revendication 1, **caractérisé en ce que** le premier récipient comprimable (1) est rempli avec un liquide jusqu'à la quantité d'air prédéterminée.

3. Système stérile selon la revendication 1 ou 2, **caractérisé en ce que** le premier récipient comprimable (1) présente une entrée (2), à laquelle conduit une conduite de collecte (4) suscepible d'être ouverte.

4. Système stérile selon l'une quelconque des revendications 1 à 3, **caractérisé en ce que** le deuxième récipient comprimable (23) présente une sortie (28), de laquelle une conduite satellite (27) conduit à un agencement de récipients satellites (24), qui comprend un ou plusieurs récipients satellites (25).

5. Système stérile selon l'une quelconque des revendications 1 à 4, **caractérisé en ce que** le premier récipient comprimable (1) est une poche de collecte pour du sang total.

6. Système stérile selon la revendication 5, **caractérisé en ce que** la poche de collecte de sang (1) est remplie avec un anticoagulant.

7. Système stérile selon l'une quelconque des revendications 1 à 6, **caractérisé en ce que** le filtre est un filtre à leucocytes (16).

8. Système stérile selon l'une quelconque des revendications 3 à 7, **caractérisé en ce que** la conduite de collecte (4) est une conduite de collecte de sang avec une aiguille (7), qui est fermée par une pince pour tuyaux souples (5).

9. Procédé pour filtrer des liquides, notamment le sang total, avec un dispositif selon l'une quelconque des revendications 1 à 8, **caractérisé par** les étapes suivantes:
(i) collecter le liquide à filtrer, notamment le sang total, dans le premier récipient comprimable (1), qui contient une quantité d'air prédéterminée ;
(ii) transférer le liquide avec la quantité d'air prédéterminée du premier récipient comprimable (1) à travers le filtre (16) et le troisième récipient comprimable (15) dans le deuxième récipient comprimable (23) ; et
(iii) transférer la quantité d'air prédéterminée du deuxième récipient comprimable (23) dans le troisième récipient comprimable (15), qui présente un volume de remplissage qui est plus petit que le volume de remplissage du premier ou du deuxième récipient comprimable.

10. Procédé selon la revendication 9, **caractérisé en ce que** le liquide à filtrer est du sang total.

11. Procédé selon la revendication 9 ou 10, **caractérisé en ce que** le filtre (16) est un filtre à leucocytes.

12. Procédé selon l'une quelconque des revendications 9 à 11, **caractérisé en ce que**, avant la collecte de sang total, le premier récipient comprimable (1) est rempli avec un anticoagulant jusqu'à la quantité d'air prédéterminée.

13. Système pour filtrer un liquide, comprenant un filtre, une conduite en tuyau souple qui relie une poche de réception à une poche de collecte et dans laquelle est monté le filtre, dans lequel le filtre est divisé en une première et une deuxième chambre, une autre poche, qui est en communication d'écoulement avec la première et la deuxième chambre respectivement par une conduite d'entrée d'air et une conduite de sortie, dans lequel il se trouve dans une des deux conduites une soupape anti-retour qui ne permet un passage de fluide qu'en direction de la première chambre du filtre, dans lequel la poche sert pour la purge d'air du système lors du transfert du liquide de la poche de réception à la poche de collecte.
